# EUROPEAN PATENT APPLICATION

(11) **EP 1 431 278 A1**
(43) Date of publication of application: **23.06.2004**
(21) Application number: 02772940.9
(22) Date of filing: 27.09.2002
(51) Int. Cl.: C07C 253/00, C07C 255/37, C07C 255/41, C07C 249/04, C07C 251/40, C07C 205/44, C07C 205/61, C07C 217/48, C07C 229/38, C07D 207/08

(54) **PROCESS FOR PRODUCING (2-NITROPHENYL)ACETONITRILE DERIVATIVE AND INTERMEDIATE THEREFOR**

(30) Priority: 28.09.2001 JP 2001299205
(71) Applicant: IHARA CHEMICAL INDUSTRY CO., LTD., Taitoh-ku, Tokyo 110-0008 (JP); KUMIAI CHEMICAL INDUSTRY CO., LTD., Tokyo 110-0008 (JP)
(72) Inventor: ABE, Tetsuy, c/o K.I Chemical Research, Iwata-gun, Shizuoka 437-1213 (JP); NAGATA, Toshihiro, c/o K.I Chemical Research, Iwata-gun, Shizuoka 437-1213 (JP); TAKABE, Fumiaki, c/o K.I Chemical Research, Iwata-gun, Shizuoka 437-1213 (JP); UMEZU, Kazuto, c/o Ihara Chem. Ind. Co., Ltd, Ihara-gun, Shizuoka 421-3306 (JP); HAMADA, Yusuke, c/o Ihara Chem. Ind. Co., Ltd, Ihara-gun, Shizuoka 421-3306 (JP)
(74) Representative: Lorenz, Eduard
(86) International application number: PCT/JP2002/010099
(87) International publication number: WO 2003/029189

(57) **Abstract**

The present invention lies in a process for producing a compound represented by the following formula [IV], which comprises the following steps (1) to (3), as well as in an intermediate therefor.
(1) A step which comprises reacting a compound represented by the following formula [I] with a compound represented by the following formula [VI] or with a mixture of a compound represented by the following formula [VII] and a compound represented by the following formula [VIII] to obtain a compound represented by the following formula [IX], and hydrolyzing the compound represented by the formula [IX] under acidic conditions to obtain a compound represented by the following formula [II].
(2) A step which comprises reacting the compound represented by the formula [II] with hydroxylamine to obtain a compound represented by the following formula [III].
(3) A step which comprises dehydrating the compound represented by the formula [III] to obtain a compound represented by the formula [IV].

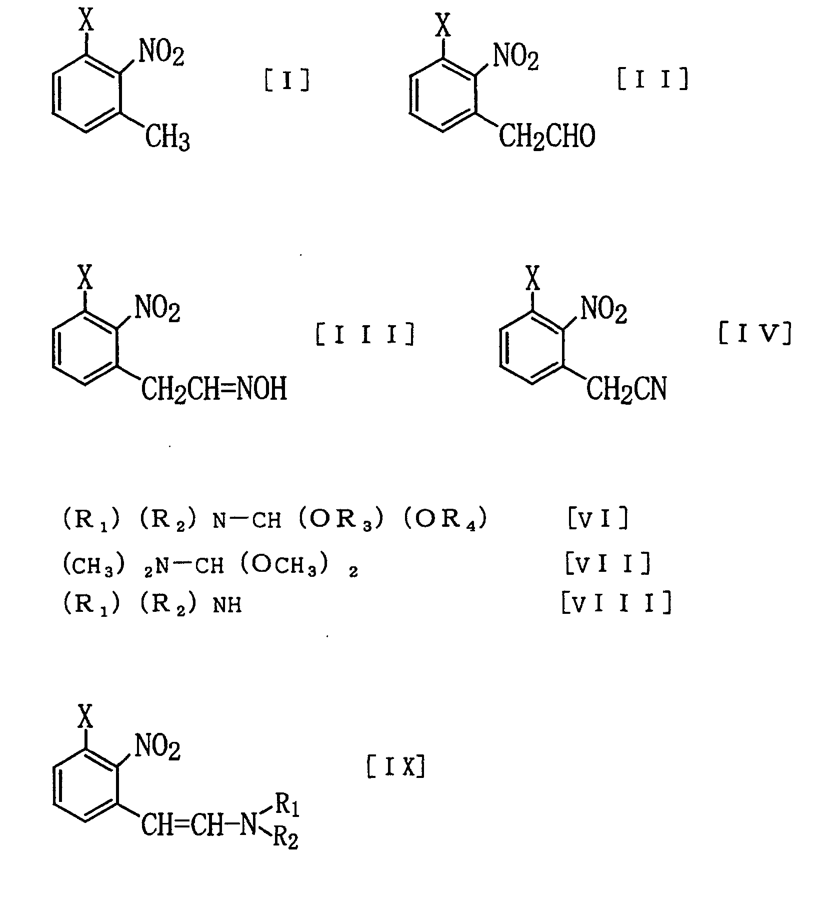

## Description

### Technical Field

The present invention relates to a novel process for producing a (2-nitro-phenyl)acetonitrile derivative which is a useful intermediate for agricultural chemicals, as well as to an intermediate used for synthesis thereof.

### Background Art

Synthesis Processes for (2-nitro-phenyl)acetonitrile derivative are described in, for example, Synthesis Vol. 5, pp. 514-515 (1987) and WO 91/15478. In each of the processes described in these literatures, a 2-nitrobenzyl bromide is reacted with a cyano compound to synthesize a (2-nitro-phenyl)acetonitrile derivative.

In the above prior art, however, the 2-nitrobenzyl bromide used as a reaction intermediate benzylates even the (2-nitro-phenyl)acetonitrile derivative which is an intended product, generating a by-product; therefore, the yield of the product has been low and the purification thereof has been difficult.

Hence, there has been sought a production process capable of producing an intended product at a small generation amount of by-product, at an easy purification of product, and at a high yield of product. The present invention responds to such a demand, and provides a novel process for producing a (2-nitro-phenyl)acetonitrile derivative, which is suitable for industrial production and an intermediate used for the production.

### Disclosure of the Invention

The present inventors made a study in order to solve the above-mentioned problems of the prior art. As a result, the present inventors found out a process capable of producing a (2-nitro-phenyl)acetonitrile derivative with no benzylation of cyano compound. A further study was made and the present invention has been completed.

That is, the present invention provides the following inventions [1] to [4], whereby the above-mentioned problems have been solved.
[1] A process for producing a (2-nitro-phenyl)acetonitrile derivative represented by the following general formula [IV] 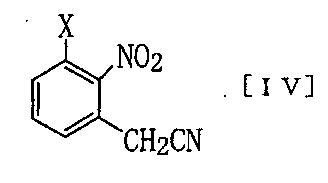 (wherein X is an alkoxymethyl group or an alkoxycarbonyl group), which comprises the following steps (A) to (C).
   (1) A step (A) which comprises reacting a 2-methylnitrobenzene derivative represented by the following general formula [I] 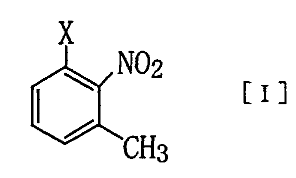 (wherein X is an alkoxymethyl group or an alkoxycarbonyl group) with an N,N-dialkylformamide dialkylacetal represented by the following general formula [VI]

      (R₁) (R₂)N-CH(OR₃) (OR₄) [VI]

      (wherein R₁ and R₂ may be the same or different, are each an alkyl group, and may form, by bonding to each other, a 5- to 6-membered ring together with the nitrogen atom; and R₃ and R₄ may be the same or different and are each an alkyl group), or with a mixture of N,N-dimethylformamide dimethylacetal represented by the following formula [VII]

      (CH₃)₂N-CH(OCH₃)₂ [VII]

      and a dialkylamine represented by the following general formula [VIII]

      (R₁) (R₂)NH [VIII]

      (wherein R₁ and R₂ have the same meanings as given above) to obtain a 2-(2-dialkylaminoethenyl)nitrobenzene derivative represented by the following general formula [IX] 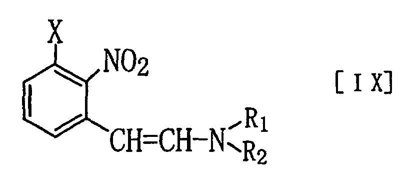 (wherein X, R₁ and R₂ have the same meanings as given above), and hydrolyzing the derivative [IX] under acidic conditions to obtain a phenylacetaldehyde derivative represented by the following general formula [II] 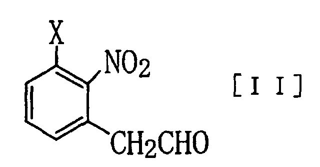 (wherein X has the same meaning as given above).
   (2) A step (B) which comprises reacting the phenylacetaldehyde derivative represented by the general formula [II], obtained in the step (A) with hydroxylamine to obtain a phenylacetoaldoxime derivative represented by the following general formula [III] 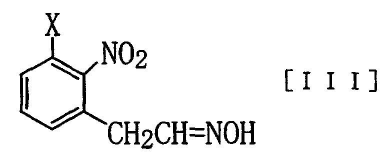 (wherein X has the same meaning as given above).
   (3) A step (C) which comprises dehydrating the phenylacetaldoxime derivative represented by the general formula [III], obtained in the step (B) to obtain a (2-nitrophenyl)acetonitrile derivative represented by the following general formula [IV] 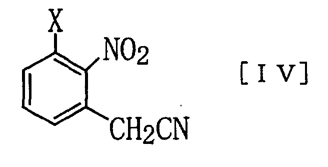 (wherein X has the same meaning as given above).
[2] A process for producing a (2-nitro-phenyl)acetonitrile derivative represented by the following general formula [IV] 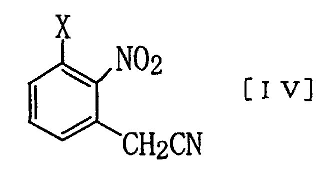 (wherein X is an alkoxymethyl group or an alkoxycarbonyl group), which comprises the following steps (D) to (E).
   (1) A step (D) which comprises reacting a 2-methylnitrobenzene derivative represented by the following general formula [I] 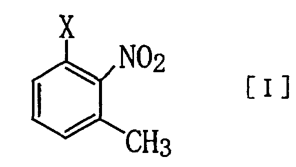 (wherein X is an alkoxymethyl group or an alkoxycarbonyl group) with an N,N-dialkylformamide dialkylacetal represented by the following general formula [VI]

      (R₁) (R₂)N-CH(OR₃) (OR₄) [VI]

      (wherein R₁ and R₂ may be the same or different, are each an alkyl group, and may form, by bonding to each other, a 5- to 6-membered ring together with the nitrogen atom; and R₃ and R₄ may be the same or different and are each an alkyl group), or with a mixture of N,N-dimethylformamide dimethylacetal represented by the following formula [VII]

      (CH₃)₂N-CH (OCH₃)₂ [VII]

      and a dialkylamine represented by the following general formula [VIII]

      (R₁) (R₂) NH [VIII]

      (wherein R₁ and R₂ have the same meanings as given above) to obtain a 2-(2-dialkylaminoethenyl)nitrobenzene derivative represented by the following general formula [IX] 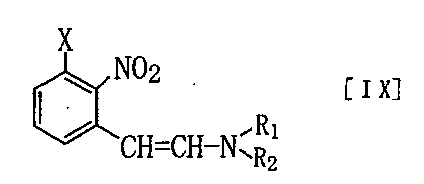 (wherein X, R₁ and R₂ have the same meanings as given above), and reacting the derivative [IX] with hydroxylamine to obtain a phenylacetaldoxime derivative represented by the following general formula [III] 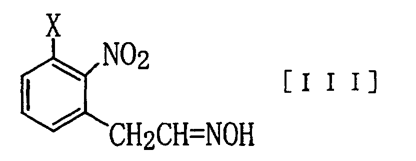 (wherein X has the same meaning as given above).
   (2) A step (E) which comprises dehydrating the phenylacetoaldoxime represented by the general formula [III], obtained in the step (D) to obtain a (2-nitro-phenyl)acetonitrile derivative represented by the following general formula [IV] 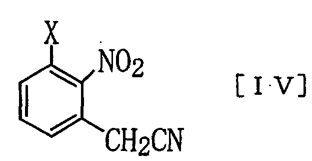 (wherein X has the same meaning as given above).
[3] A process for producing a phenylacetoaldoxime derivative represented by the following general formula [III] 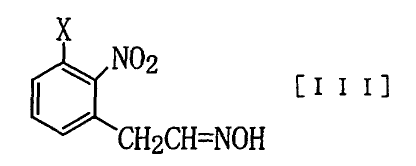 (wherein X is an alkoxymethyl group or an alkoxycarbonyl group), which comprises reacting a 2-(2-dialkylaminoethenyl)nitrobenzene derivative represented by the following general formula [IX] 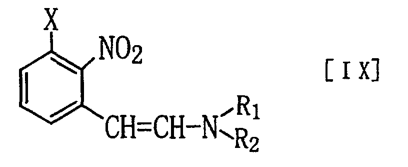 (wherein X has the same meaning as given above; and R₁ and R₂ may be the same or different, are each an alkyl group, and may form, by bonding to each other, a 5- to 6-membered ring together with the nitrogen atom) with hydroxylamine.
[4] A nitrobenzene derivative represented by the following general formula [V] 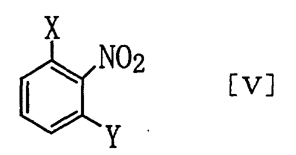 [wherein X is an alkoxymethyl group or an alkoxycarbonyl group; Y is a methyl group, a 2-dialkylaminoethenyl group represented by the following general formula [XI]

   -CH=N(R₁) (R₂) [XI]

   (wherein R₁ and R₂ may be the same or different, are each an alkyl group, and may form, by bonding to each other, a 5- to 6-membered ring together with the nitrogen atom), a formylmethyl group or a 2-hydroxyiminoethyl group; when Y is a methyl group or a 2-dialkylaminoethenyl group represented by the general formula [XI], X is a methoxymethyl group].

### Best Mode for Carrying Out the Invention

First, description is made on the process [1] of the present invention.

The present process [1] is a process for producing a (2-nitro-phenyl)acetonitrile derivative represented by the following general formula [IV] 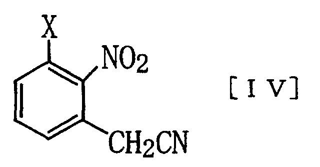 (wherein X is an alkoxymethyl group or an alkoxycarbonyl group), which comprises the above-mentioned steps (A) to (C). Each product obtained in each of the steps (A) to (C) or in each reaction of each step need not be isolated or purified for use in next step or next reaction; and, as necessary, the steps (A) to (C) or the reactions of each step may be conducted continuously in one-pot.

### (1) The step (A) is represented by the following scheme.

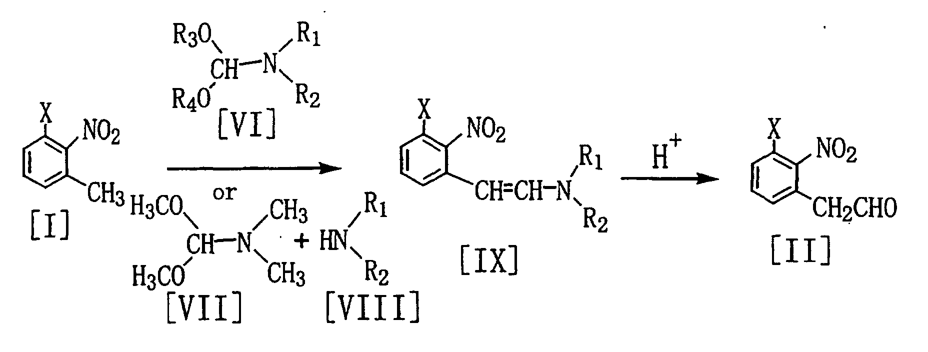

(wherein X has the same meaning as given above; R₁ and R₂ may be the same or different, are each an alkyl group, and may form, by bonding to each other, a 5- to 6-membered ring together with the nitrogen atom; and R₃ and R₄ may be the same or different and are each an alkyl group).

The 2-methylnitrobenzene derivative represented by the following general formula [I] 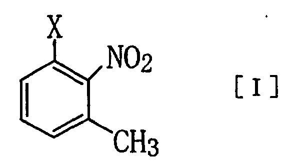 (wherein X is an alkoxymethyl group or an alkoxycarbonyl group), which is a raw material in the step (A), may be a compound wherein X is a straight chain or branched chain alkoxymethyl group having 1 to 6 carbon atoms (hereinafter, the carbon atoms are abbreviated to, for example, "C1 to C6" in in this case) [i.e. (C1 to C6 alkoxy)methyl group] or a straight chain or branched chain (C1 to C6 alkoxy)carbonyl group. As the (C1 to C6 alkoxy)methyl group, there can be mentioned, for example, methoxymethyl group, ethoxymethyl group, n-propoxymethyl group, isopropoxymethyl group, n-butoxymethyl group, n-pentyloxymethyl group and n-hexyloxymethyl group. As the (C1 to C6 alkoxy)carbonyl group, there can be mentioned, for example, methoxycarbonyl group, ethoxycarbonyl group, n-propoxycarbonyl group, isopropoxycarbonyl group, n-butoxycarbonyl group, n-pentyloxycarbonyl group and n-hexyloxycarbonyl group.

As specific examples of the 2-methylnitrobenzene derivative, there can be mentioned, for example, 3-methoxymethyl-2-nitrotoluene, 3-ethoxymethyl-2-nitrotoluene, 3-n-propoxymethyl-2-nitrotoluene, 3-isopropoxymethyl-2-nitrotoluene, 3-n-butoxymethyl-2-nitrotoluene, 3-n-pentyloxymethyl-2-nitrotoluene, 3-n-hexyloxymethyl-2-nitrotoluene, methyl 3-methyl-2-nitrobenzoate, ethyl 3-methyl-2-nitrobenzoate, n-propyl 3-methyl-2-nitrobenzoate, n-butyl 3-methyl-2-nitrobenzoate, n-pentyl 3-methyl-2-nitrobenzoate and n-hexyl 3-methyl-2-nitrobenzoate.

Of these 2-methylnitrobenzene derivatives represented by the general formula [I], a compound wherein X is an alkoxymethyl group, can be produced, for example, by reducing a 3-methyl-2-nitrobenzoate (typically a methyl ester) or a 3-methyl-2-nitrobenzoyl halide (typically a chloride) to obtain 3-methyl-2-nitrobenzyl alcohol and further alkylating the alcohol in accordance with, for example, the method described in Tetrahedron Letter, Vol. 30, pp. 47-50 (1989).

A compound wherein X is an alkoxycarbonyl group, is a known compound or can be produced, for example, by 2-nitrating 3-methylbenzoic acid in accordance with the method described in Bull. Chem. Soc. Jpn., Vol. 60, p. 3659 (1987), converting the resulting compound into an acid halide with thionyl chloride or the like according to an ordinary method, and further reacting a 3-methyl-2-nitrobenzoyl halide (e.g. 3-methyl-2-nitrobenzoic chloride) with a corresponding alcohol in accordance with a known method, for example, the method described in Chemical Reviews, Vol. 52, p. 237 (1953) to give rise to esterification.

As the corresponding alcohol, there can be mentioned, for example, C1 to C6 straight chain or branched chain alcohols; specifically, for example, methanol, ethanol, n-propanol, i-propanol, n-butanol, i-butanol, sec-butanol, tert-butanol, n-pentanol and n-hexanol.

As the N,N-dialkylformamide dialkylacetal represented by the general formula [VI], which is a raw material in the step (A), there can be mentioned, for example, N,N-di(C1 to C6 alkyl)formamide di(C1 to C6 alkyl)acetals, specifically, N,N-dimethylformamide dimethylacetal, N,N-dimethylformamide diethylacetal, N,N-diethylformamide dimethylacetal, N,N-dipropylformamide dimethylacetal, N-methyl-N-ethyl-formamide dimethylacetal etc.; and compounds wherein R₁ and R₂ form a 5- to 6-membered ring together with the nitrogen atom to which R₁ and R₂ bond, specifically a compound R₁ and R₂ form, for example, a pyrrolidine ring.

It is possible to use, in place of the N,N-dialkylformamide dialkylacetal, an N,N-dimethylformamide dimethylacetal represented by the formula [VII] and a dialkylamine represented by the general formula [VIII]. As the dialkylamine represented by the general formula [VIII], there can be mentioned, for example, di(C1 to C6 alkyl)amines, specifically dimethylamine, diethylamine, dipropylamine, dibutylamine and methylethylamine; and compounds wherein R₁ and R₂ form a 5- to 6-membered ring together with the nitrogen atom, specifically, pyrrolidine, etc.

With respect to the amounts of the reagents used in the above reaction of the step (A), the amounts of the compounds represented by the general formulas [VI] to [VIII] are each 1 to 10 equivalents, preferably 1 to 3 equivalents relative to 1 equivalent of the 2-methylnitrobenzene derivative represented by the general formula [I].

As the solvent usable in the step (A), there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and he like; aromatic hydrocarbons such as benzene, toluene, xylene and the like; and mixtures thereof. The use amount of the solvent is, for example, 10,000 ml or less, preferably 1,000 ml or less relative to 1 mole of the 2-methylnitrobenzene derivative represented by the general formula [I].

As the acid used in the step (A) to hydrolyze, under acidic conditions, the 2-(2-dialkylaminoethenyl)nitrobenzene derivative represented by the general formula [IX], there can be mentioned mineral acids such as hydrochloric acid, sulfuric acid and the like; and organic acids such as acetic acid, methanesulfonic acid, p-toluenesulfonic acid and the like. The amount of the acid used in the reaction is 1 to 20 equivalents, preferably 1 to 3 equivalents relative to 1 equivalent of the 2-methylnitrobenzene derivative represented by the general formula [I].

The reactions of the step (A) are each conducted at a desired temperature ranging from -10°C to the reflux temperature of the reaction system, preferably, for example, at a temperature ranging from 0°C to 130°C. Each reaction is complete in 0.5 to 24 hours although the time differs depending upon the compounds used.

Successively, description is made on the step (B).

### (2) The step (B) is represented by the following scheme.

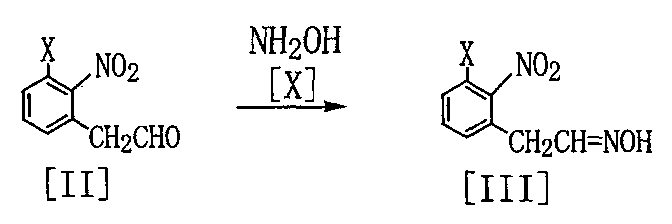

(wherein X has the same meaning as given above).

The phenylacetoaldoxime derivative represented by the general formula [III], which is an intended product in the step (B), can be produced by reacting the phenylacetoaldehyde derivative represented by the general formula [II], obtained in the step (A) with a hydroxylamine represented by the formula [X] or a mineral acid salt thereof in the presence or absence of a solvent (preferably in an appropriate solvent) in the presence or absence of a base.

The amount of the hydroxylamine represented by the formula [X] or its mineral acid salt (e.g. hydrochloride, nitrate or sulfate), used in the step (B) is 1 to 2 equivalents, preferably 1 to 1.2 equivalents relative to 1 equivalent of the phenylacetoaldehyde derivative represented by the general formula [II].

As the base used in the step (B), there can be mentioned, for example, metal hydrides such as sodium hydride and the like; alkali metal amides such as sodium amide, lithium diisopropylamide and the like; organic bases such as pyridine, triethylamine, 1,8-diazabicyclo[5.4.0]-7-undecene and the like; alkali metal hydroxides such as sodium hydroxide, potassium hydroxide and the like; alkaline earth metal hydroxides such as calcium hydroxide, magnesium hydroxide and the like; alkali metal carbonates such as sodium carbonate, potassium carbonate and the like; alkali metal bicarbonates such as sodium hydrogencarbonate, potassium hydrogencarbonate and the like; alkali metal carboxylates such as sodium acetate, potassium formate and the like; and metal salts of alcohols, such as sodium methoxide, potassium tert-butoxide and the like. The use amount of the base is, for example, 1 to 2 moles, preferably 1 to 1.2 moles relative to 1 mole of the phenylacetoaldehyde derivative represented by the general formula [II].

As the solvent usable in the step (B), there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and he like; aromatic hydrocarbons such as benzene, toluene, xylene and the like; alcohols such as methanol, ethanol, propanol, isopropanol, butanol, tert-butanol and the like; nitriles such as acetonitrile and the like; water; and mixtures thereof. The use amount of the solvent is, for example, 10,000 ml or less, preferably 1,000 ml or less relative to 1 mole of the phenylacetaldehyde derivative represented by the general formula [II].

The reaction of the step (B) is conducted at a desired temperature ranging from 0°C to the reflux temperature of the reaction system, preferably, for example, at a temperature ranging from 20°C to 70°C. The reaction is complete in 1 to 72 hours although the time differs depending upon the compounds used.

Successively, description is made on the step (C).

### (3) The step (C) is represented by the following scheme.

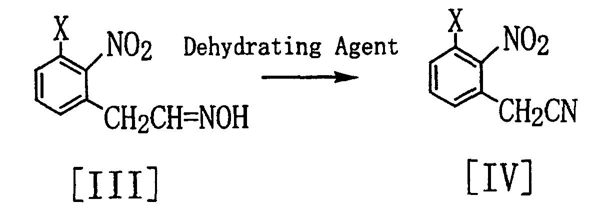

(wherein X has the same meaning as given above).

The phenylacetonitrile derivative represented by the general formula [IV], which is an intended product of the step (C), can be produced by reacting the phenylacetoaldoxime derivative represented by the general formula [III], obtained in the step (B), with a dehydrating agent in the presence or absence of a solvent (preferably in an appropriate solvent).

As the dehydrating agent used in the step (C), there can be mentioned those such as acid halides (e.g. acetyl chloride, phosgene, thionyl chloride and phosphorus oxychloride), acid anhydrides (e.g. trifluoroacetic anhydride and acetic anhydride), carbonyl diimidazole, dicyclohexane diimide) and the like.

As to the amount of the reagent used in the step (C), the amount of the dehydrating agent is 1 to 3 equivalents relative to 1 equivalent of the phenylacetaldoxime derivative represented by the general formula [III].

As the solvent usable in the step (C), there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and the like; aromatic hydrocarbons such as benzene, toluene, xylene and the like; esters such as ethyl acetate, isopropyl acetate and the like; ketones such as acetone, 2-butanone and the like; nitriles such as acetonitrile and the like; and mixtures thereof. The use amount of the solvent is, for example, 10,000 ml or less, preferably 1,000 ml or less relative to 1 mole of the phenylacetoaldoxime derivative represented by the general formula [III].

The reaction of the step (C) is conducted at a desired temperature ranging from 0°C to the reflux temperature of the reaction system, preferably, for example, at a temperature ranging from 70°C to 150°C. The reaction is complete in 1 to 72 hours although the time differs depending upon the compounds used.

Successively, description is made, for reference, on a step which comprises reducing a phenylacetonitrile derivative represented by the general formula [IV], which is obtained in the step (C) and wherein X is an alkoxycarbonyl group, to convert it into 2-nitro-3-cyanomethyl-benzyl alcohol and then producing, from the alcohol, a (2-nitro-3-alkoxymethyl-phenyl)acetonitrile.

This step for reference is represented by the following scheme. 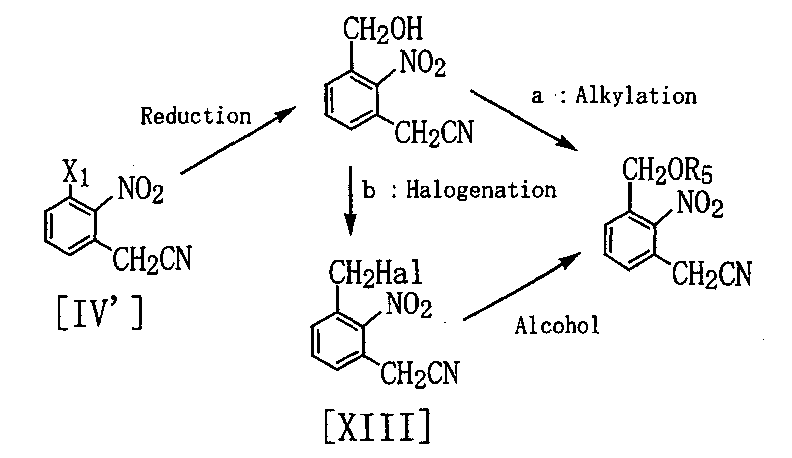 (wherein X₁ is an alkoxycarbonyl group, Hal is a halogen atom, and R₅ is an alkyl group).

This step comprises, as shown in the above scheme, reducing a (3-alkoxycarbonyl-2-nitro-phenyl)acetonitrile derivative represented by the general formula [IV'] [i.e. a (2-nitro-phenyl)acetonitrile derivative represented by the general formula [IV], which is obtained in the step (C) and wherein X is an alkoxycarbonyl group], to convert it into 2-nitro-3-cyanomethyl-benzyl alcohol and then producing, from the alcohol, a (2-nitro-3-alkoxymethyl-phenyl)acetonitrile according to a method a or b. Here, a or b is the following reaction.
a: 2-Nitro-3-cyanomethyl-benzyl alcohol is reacted with an alkylating agent.
b: 2-Nitro-3-cyanomethyl-benzyl alcohol is halogenated to obtain a 2-nitro-3-cyanomethyl-benzyl halide derivative represented by the following general formula [XIII] 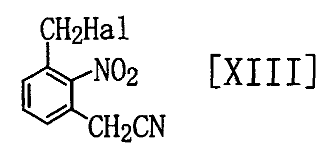 (wherein Hal is a halogen atom) and the derivative is reacted with an alcohol.

First, description is made on the reduction of the present step.

In the present step, the reduction of the (3-alkoxycarbonyl-2-nitro-phenyl)acetonitrile derivative represented by the general formula [IV'] is conducted using a reducing agent. The reducing agent may be any reducing agent as long as it can reduce the alkoxycarbonyl group into a hydroxymethyl group without reducing the nitrile group, and can be exemplified by a combination of sodium borohydride and methanol. When the combination of sodium borohydride and methanol is used as the reducing agent, sodium borohydride is used in an amount of, for example, 1.0 to 3.0 moles, preferably 1.0 to 1.5 moles and methanol is used in an amount of, for example, 1.0 to 20 moles, preferably 2 to 10 moles, all relative to 1 mole of the (3-alkoxycarbonyl-2-nitro-phenyl)acetonitrile represented by the general formula [IV'].

The reduction of the present step is ordinarily conducted in a solvent. As the usable solvent, there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and the like; aromatic hydrocarbons such as benzene, toluene, xylene and the like; esters such as ethyl acetate, isopropyl acetate and the like; ketones such as acetone, 2-butanone and the like; nitriles such as acetonitrile and the like; and mixtures thereof. The use amount of the solvent is, for example, 100 to 5,000 ml, preferably 300 to 1,000 mol relative to 1 mole of the (3-alkoxycarbonyl-2-nitro-phenyl)acetonitrile represented by the general formula [IV'].

The reduction of the present step is conducted at a desired temperature ranging from 40°C to the reflux temperature of the reaction system, preferably at a temperature ranging from 50°C to the reflux temperature of the reaction system. When a combination of sodium borohydride and methanol is used as the reducing agent, methanol is added dropwise under refluxing. The reaction is complete in 1 to 72 hours although the time differs depending upon the compounds used.

Next, description is made on the method a of the present step.

In the method a of the present step, 2-nitro-3-cyanomethyl-benzyl alcohol is reacted with an alkylating agent.

In the method a of the present step, the alkylation of 2-nitro-3-cyanomethyl-benzyl alcohol is conducted using an alkylating agent. The alkylating agent may be any alkylating agent as long as it can alkylate the benzyl alcohol into an alkoxymethyl group. It can be exemplified by di(C1 to C6 alkyl) sulfates such as dimethyl sulfate, diethyl sulfate and the like; and C1 to C6 alkyl halides such as methyl iodide, ethyl bromide and the like. The use amount of the alkylating agent is, for example, 1.0 to 2.0 moles, preferably 1.0 to 1.5 moles per 1 mole of 2-nitro-3-cyanomethyl-benzyl alcohol.

In the method a of the present step, the alkylation is ordinarily conducted in a solvent. As the usable solvent, there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and the like; aromatic hydrocarbons such as benzene, toluene, xylene and the like; esters such as ethyl acetate, isopropyl acetate and the like; ketones such as acetone, 2-butanone and the like; nitriles such as acetonitrile and the like; and mixtures thereof. The use amount of the solvent is, for example, 500 to 5,000 ml, preferably 1,000 to 3,000 ml relative to 1 mole of 2-nitro-3-cyanomethyl-benzyl alcohol.

In the method a of the present step, the alkylation is conducted at a desired temperature ranging from 10°C to the reflux temperature of the reaction system, preferably at a temperature ranging from 20°C to 40°C. The reaction is complete in 1 to 72 hours although the time differs depending upon the compounds used.

Successively, description is made on the method b of the present step.

In the method b of the present step, 2-nitro-3-cyanomethyl-benzyl alcohol is halogenated to obtain a 2-nitro-3-cyanomethyl-benzyl halide derivative represented by the following general formula [XIII] 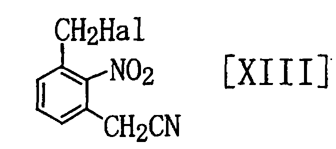 (wherein Hal is a halogen atom) and then the derivative is reacted with an alcohol.

In the method b of the present step, the halogenation of 2-nitro-3-cyanomethyl-benzyl alcohol is conducted using a halogenating agent. The halogenating agent may be any halogenating agent as long as it can halogenate hydroxymethyl group into halogenomethyl group. It can be exemplified by phosphorus trihalides (e.g. phosphorus trichloride and phosphorus tribromide), thionyl halides (e.g. thionyl chloride) and phosphorus pentahalides (e.g. phosphorus pentachloride and phosphorus pentabromide). The use amount of the halogenating agent is, for example, 1.0 to 3.0 moles, preferably 1.0 to 1.5 moles per 1 mole of 2-nitro-3-cyanomethyl-benzyl alcohol.

In the method b of the present step, the halogenation is ordinarily conducted in a solvent. As the usable solvent, there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and the like; aromatic hydrocarbons such as benzene, toluene, xylene and the like; esters such as ethyl acetate, isopropyl acetate and the like; ketones such as acetone, 2-butanone and the like; nitriles such as acetonitrile and the like; and mixtures thereof. The use amount of the solvent is, for example, 500 to 5,000 ml, preferably 1,000 to 3,000 ml relative to 1 mole of 2-nitro-3-cyanomethyl-benzyl alcohol.

In the method b of the present step, the halogenation is conducted at a desired temperature ranging from 10°C to the reflux temperature of the reaction system, preferably at a temperature ranging from 20°C to 40°C. The reaction is complete in 1 to 72 hours although the time differs depending upon the compounds used.

In the method b of the present step, the alcohol used in the reaction of a 2-nitro-3-cyanomethyl-benzyl halide derivative represented by the general formula [XIII] and an alcohol may be a straight chain or branched chain C1 to C6 alcohol, specifically methanol, ethanol, n-propanol, i-propanol, n-butanol, i-butanol, sec-butanol, tert-butanol, n-pentanol, n-hexanol or the like. The amount of the C1 to C6 alcohol used in the reaction is 1.0 to 20 equivalents, preferably 1.0 to 10 equivalents relative to 1 equivalent of the 2-nitro-3-cyanomethyl-benzyl halide derivative represented by the general formula [XIII].

In the method b of the present step, the reaction of the 2-nitro-3-cyanomethyl-benzyl halide derivative represented by the general formula [XIII] with the alcohol may be conducted in a solvent. As the usable solvent, there can be mentioned, for example, ethers such as dioxane, tetrahydrofuran (THF) and the like; halogenated hydrocarbons such as dichloroethane, carbon tetrachloride, chlorobenzene, dichlorobenzene and the like; amides such as N,N-dimethylacetamide, N,N-dimethylformamide (DMF), N-methyl-2-pyrrolidinone and the like; sulfur compounds such as dimethyl sulfoxide (DMSO), sulfolane and the like; aromatic hydrocarbons such as benzene, toluene, xylene and the like; esters such as ethyl acetate, isopropyl acetate and the like; ketones such as acetone, 2-butanone and the like; nitriles such as acetonitrile and the like; and mixtures thereof. The use amount of the solvent is, for example, 5,000 ml or less, preferably 1,000 ml or less relative to 1 mole of the 2-nitro-3-cyanomethyl-benzyl halide derivative represented by the general formula [XIII].

In the method b of the present step, the reaction with the alcohol is conducted at a desired temperature ranging from 50°C to 100°C, preferably at a temperature ranging from 60°C to 90°C. The reaction is complete in 1 to 72 hours although the time differs depending upon the compounds used.

The (2-nitro-phenyl)acetonitrile derivative represented by the general formula [IV] obtained in the present process, typified by (3-methoxymethyl-2-nitro-phenyl)acetonitrile can be derived into a raw material for agricultural chemicals described in WO 00/06553, by, as shown in, for example, the following scheme, reacting the compound with 2-methanesulfonyl-4,6-dimethoxypyrimidine and then subjecting the reaction product to oxidative decyanation using, for example, m-chloroperbenzoic acid or hydrogen peroxide.

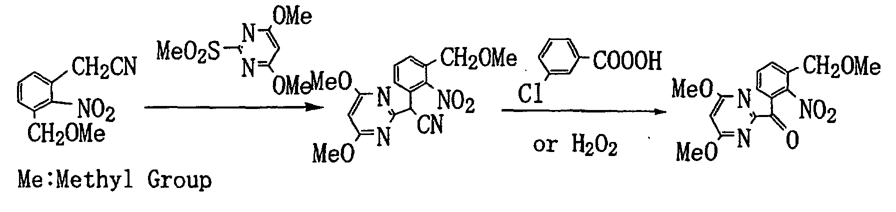

Meanwhile, the present process [2] is a process for producing a (2-nitro-phenyl)acetonitrile derivative represented by the following general formula [IV] 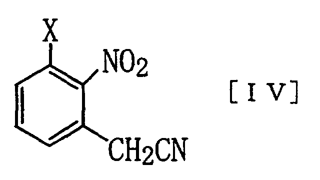 (wherein X is an alkoxymethyl group or an alkoxycarbonyl group), which comprises steps (D) and (E). Each product obtained in each of the steps (D) and (E) or in each reaction of each step need not be isolated or purified for use in next step or next reaction; and, as necessary, the steps (D) and (E) or the reactions of these steps may be conducted continuously in one-pot.

(1) The step (D) comprises reacting a 2-methylnitrobenzene derivative represented by the above-shown general formula [I] with an N,N-dialkylformamide dialkylacetal represented by the above-shown general formula [VI] or with a mixture of N,N-dimethylformamide dimethylacetal represented by the formula [VII] and a dialkylamine represented by the general formula [VIII] to obtain a 2-(2-dialkylaminoethenyl)nitrobenzene derivative represented by the general formula [IX], and reacting the derivative [IX] with hydroxylamine to obtain a phenylacetoaldoxime derivative represented by the following general formula [III] 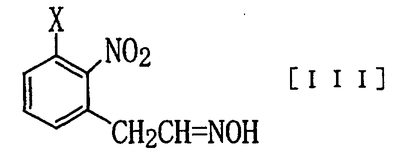 (wherein X has the same meaning as given above). In the step (D), the 2-(2-dialkylaminoethenyl)nitrobenzene derivative represented by the general formula [IX] is directly reacted with hydroxylamine without via the phenylacetoaldehyde derivative represented by the general formula [II] as in the step (A) of the present process [1], to obtain a phenylacetoaldoxime derivative represented by the general formula [III].

The hydroxylamine (or its mineral acid salt) and its amount, used in the step (D) are the same as in the step (A) of the present process [1].

The step (D) can be conducted in the absence of a solvent but may be conducted in the presence of a solvent. The usable solvent is a solvent inert to the reaction. As such a solvent, there can be mentioned, for example, aprotic solvents such as aromatic hydrocarbon type solvent (e.g. toluene, xylene, chlorobenzene or o-dichlorobenzene), aliphatic hydrocarbon type solvent (e.g. n-hexane, n-heptane or n-decane) and ether type solvent (e.g. dipropyl ether, diisopropyl ether or dibutyl ether); and water. These solvents can be used singly or in admixture of two or more kinds.

The solvent is preferably an aprotic solvent which is easy to separate from water in, for example, the treatment after reaction and is low in load for waste water removal. It is possible to use water and other solvent separable from water (for example, an aprotic solvent selected from aromatic hydrocarbon type solvents, aliphatic hydrocarbon type solvents, ether type solvents, etc.) and conduct the reactions in a two-phase solvent system.

The use amount of the solvent can be any as long as sufficient stirring of the reaction system is secured. The use amount is, for example, 5,000 ml or less, preferably 1,000 ml or less relative to 1 mole of the 2-(2-dialkylaminoethenyl)nitrobenzene derivative represented by the general formula [IX], in view of the reaction rate, etc.

The reactions of the step (D) are conducted at a desired temperature ranging from -10°C to the reflux temperature of the reaction system, preferably at a temperature ranging from 0 to 60°C. The reactions can be conducted in 0.1 to 24 hours. The reactions can be conducted under normal pressure and no pressurization is required ordinarily.

(2) The step (E) comprises dehydrating the phenylacetoaldoxime derivative represented by the general formula [III], obtained in the above step (D) to obtain a (2-nitro-phenyl)acetonitrile derivative represented by the following general formula [IV] 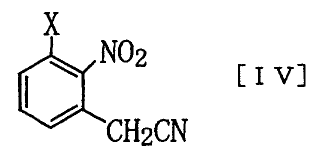 (wherein X has the same meaning as given above). This step is the same as the step (C) of the present process [1] described above.

The present process [3] produces a phenylacetoaldoxime derivative represented by the following general formula [III] 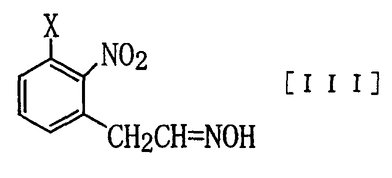 (wherein X is an alkoxymethyl group or an alkoxycarbonyl group), by, according to the present process [2], reacting a 2-(2-dialkylaminoethenyl)nitrobenzene derivative represented by the following general formula [IX] 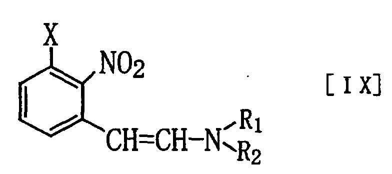 (wherein X has the same meaning as given above; and R₁ and R₂ may be the same or different, are each an alkyl group, and may form, by bonding to each other, a 5- to 6-membered ring together with the nitrogen atom) with hydroxylamine.

The present invention [4] lies in an intermediate represented by the following general formula [V], used in the present production process; 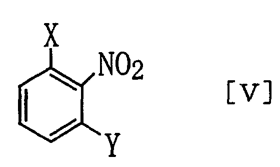 and provides a novel compound (a nitrobenzene derivative) represented by the above general formula [V].

Therefore, in the general formula [V], X is an alkoxymethyl group or an alkoxycarbonyl group; Y is a methyl group, a 2-dialkylaminoethenyl group represented by the following general formula [XI]

-CH=N (R₁) (R₂) [XI]

(wherein R₁ and R₂ may be the same or different, are each an alkyl group, and may form, by bonding to each other, a 5- to 6-membered ring together with the nitrogen atom), a formylmethyl group or a 2-hydroxyiminoethyl group.

When Y is a methyl group or a 2-dialkylaminoethenyl group represented by the general formula [XI], X is a methoxymethyl group.

Next, the present process and the process for producing the present compound are described specifically by way of Examples.

### <Reference Example 1> Synthesis of 3-methoxymethyl-2-nitrotoluene

Sodium borohydride (19.4 g, 0.51 mol) was added to a tetrahydrofuran solution (250 ml) containing methyl 3-methyl-2-nitrobenzoate (100 g, 0.51 mol). To the mixture being refluxed was dropwise added methanol (49.2 g, 1.54 mol) slowly in 30 minutes. After the dropwise addition, the mixture was refluxed for 1 hour until there was no foaming. After cooling, the mixture was poured into water and extraction with diisopropyl ether was conducted. The organic layer was washed with water, dried over anhydrous magnesium sulfate, and concentrated to obtain light brown crystals of 3-hydroxymethyl-2-nitrotoluene (yield: 80.3 g and 94%, melting point: 41 to 42°C). The crystals were made into a tetrahydrofuran solution (350 ml). A sodium hydroxide powder (26.9 g, 0.67 mol) was added to the solution, followed by stirring at room temperature for 1 hour. After a white solid appeared, dimethyl sulfate (90.9 g, 0.72 mol) was dropwise added at 30 °C or less in 30 minutes. During the dropwise addition, the system became homogeneous once and then became agar-like. The system was stirred for 2 days in that state and then poured into water. Thereto was added ammonia water (100 g). Stirring was conducted for 1 hour, after which extraction with ethyl acetate was made. The organic layer was washed with water, dried over anhydrous magnesium sulfate, and concentrated to obtain 3-methoxymethyl-2-nitrotoluene of liquid state (87.0 g, 100%, purity: 96%).

### Confirmed data

- ¹H-NMR (CDCl₃) δ:: 7.41-7.23 (3H,m), 4.49 (2H,s), 3.37 (3H,d), 2.35 (3H,s) ppm

### <Example 1>

### (A) Synthesis of 3-methoxymethyl-2-nitro-phenylacetoaldehyde

A mixed solution of 3-methoxymethyl-2-nitrotoluene (15.0 g, 82.8 mmol), pyrrolidine (13.0 g, 0.18 mol), N,N-dimethylformamide dimethylacetal (19.7 g, 0.17 mol) and N,N-dimethylformamide (6.05 g, 82.8 mmol) was heated for 1 hour up to 125°C (inside temperature) while removing low-boiling products using a Dean-Stark trap. The reaction mixture was concentrated to obtain 3-methoxymethyl-2-(2-(pyrrolidin-1-yl)ethenyl}nitrobenzene (22.7 g, yield: 100%).

### Confirmed data

m/Z: 262 (M⁺), 145, 132, 112 (base peak), 105, 70

The obtained 3-methoxymethyl-2-{2-(pyrrolidin-1-yl)ethenyl}nitrobenzene (22.7 g, 82.8 mmol) was added to a 10% aqueous hydrochloric acid solution (300 ml) with icecooling. Then, diisopropyl ether (300 ml) was added. The mixture was stirred at room temperature overnight, followed by extraction with diisopropyl ether. The organic layer was washed with diluted hydrochloric acid and with water, dried over anhydrous magnesium sulfate, and concentrated to obtain 3-methoxymethyl-2-nitro-phenylacetoaldehyde of liquid state (yield: 10.6 g and 61%, purity: 94%).

### Confirmed data

- ¹H-NMR (CDCl₃) δ :: 9.76 (1H,tri), 7.57-7.25 (3H,m), 4.57 (2H,s), 3.80 (2H,s), 3.40 (3H,s) ppm

### (B) Synthesis of 3-methoxymethyl-2-nitro-phenylacetoaldoxime

To a methanol solution (13 ml) of 3-methoxymethyl-2-nitro-phenylacetoaldehyde (5.60 g, 26.8 mmol) were added sodium acetate (2.05 g, 29.5 mmol) and hydroxylamine hydrochloride (2.44 g, 34.8 mmol) in this order. The mixture was stirred at room temperature for 30 minutes and then concentrated. The concentrate was poured into water and extraction with ethyl acetate was made. The organic layer was washed with water, dried over anhydrous magnesium sulfate, and concentrated to obtain light yellow crystals of 3-methoxymethyl-2-nitro-phenylacetoaldoxime {GC-MS: m/Z = 224 (M⁺), 206 (M⁺-H₂O)} (yield: 6.06 g and 100%, purity: 92%, melting point: 92 to 95°C).

### Confirmed data

- ¹H-NMR (CDCl₃) δ:: 7.47-7.34 (3H,m), 6.87 (1H,tri), 4.52 (2H,s), 3.73 (2H,d), 3.39 (3H,s) ppm

### (C) Synthesis of 3-methoxymethyl-2-nitro-phenylacetonitrile

3-Methoxymethyl-2-nitro-phenylacetoaldoxime (2.24 g, 10.0 mmol) was dissolved in acetic anhydride (1.53 g, 15.0 mmol). The solution was stirred at 120°C for 30 minutes. The solution was cooled and poured into water, followed by extraction with ethyl acetate. The organic layer was washed with an aqueous sodium bicarbonate solution and then with water, dried over anhydrous magnesium sulfate, and concentrated to obtain light brown to brown crystals of 3-methoxymethyl-2-nitro-phenylacetonitrile (yield: 2.14 g and 100%, purity: 91%, melting point: 34 to 36°C).

### Confirmed data

- ¹H-NMR (CDCl₃) δ:: 7.61-7.59 (3H,m), 4.55 (2H,s), 3.86 (2H,d), 3.40 (3H,s) ppm

### <Example 2>

### (A) Synthesis of methyl 3-formylmethyl-2-nitrobenzoate

An N,N-dimethylformamide solution (50 ml) containing methyl 3-methyl-2-nitrobenzoate (19.5 g, 0.10 mol) and N,N-dimethylformamide dimethylacetal (23.8 g, 0.20 mol) was refluxed for 5 hours. A Dean-Stark trap was attached and refluxing was conducted for a further 5 hours without distilling off low-boiling products. N,N-dimethylformamide dimethylacetal (11.9 g, 0.10 mol) was added and refluxing was conducted for 5 hours. After the disappearance of methyl 3-methyl-2-nitrobenzoate was confirmed and the reaction mixture was cooled, the mixture was poured into a 10% aqueous HCl solution (300 ml), followed by stirring at room temperature for 1 hour. Extraction with ethyl acetate was conducted. The aqueous layer was made alkaline with an aqueous sodium bicarbonate solution and then extraction with ethyl acetate was conducted. The organic layers obtained were combined, washed with water, dried over anhydrous magnesium sulfate, and concentrated to obtain methyl 3-formylmethyl-2-nitrobenzoate of liquid state (yield: 21.0 g and 90%).

### Confirmed data

- ¹H-NMR (CDCl₃) δ:: 9.75 (1H,d), 7.95 (1H,d-d), 7.60 (1H,tri), 7.51 (1H,d-d), 3.91 (3H,s), 3.78 (2H,s) ppm

### (B) Synthesis of methyl 3-(2-hydroxyimino)ethyl-2-nitrobenzoate

To a methanol solution (50 ml) of methyl 3-formylmethyl-2-nitrobenzoate (21.0 g, 90 mmol) were added, at room temperature, sodium acetate (9.10 g, 0.13 mol) and hydroxylamine hydrochloride (7.64 g, 0.11 mol) in this order. Stirring was made at room temperature for 30 minutes. The reaction mixture was concentrated; the concentrate was poured into water; and extraction with ethyl acetate was conducted. The organic layer was washed with water, dried over anhydrous magnesium sulfate, and concentrated to obtain light brown to brown crystals of methyl 3-(2-hydroxyimino)ethyl-2-nitrobenzoate (yield: 21.0 g and 98%, melting point: 103 to 106°C).

### Confirmed data

- ¹H-NMR (CDCl₃) δ:: 7.99 (1H,br), 7.93 (1H,d-d), 7.61 (1H,d-d), 7.54 (1H,tri), 6.85 (1H,tri), 3.91 (3H,s), 3.72 (2H,d) ppm

### (C) Synthesis of methyl 3-cyanomethyl-2-nitrobenzoate

Methyl 3-(2-hydroxyimino)ethyl-2-nitrobenzoate (21.0 g, 88 mmol) was dissolved in acetic anhydride (15.3 g, 0.15 mol). The solution was stirred at 120°C for 30 minutes. After cooling, the reaction mixture was poured into water and extraction with ethyl acetate was made. The organic layer was washed with an aqueous sodium bicarbonate solution and with water, dried over anhydrous magnesium sulfate, and concentrated to obtain a crude product. The crude product was purified using a silica gel column (ethyl acetate:hexane = 1:2 to 1:1), followed by washing with diisopropyl ether, to obtain light brown crystals of methyl 3-cyanomethyl-2-nitrobenzoate (yield: 15.4 g and 80%, melting point: 77 to 80°C).

### Confirmed data:

- ¹H-NMR (CDCl₃) δ:: 8.01 (1H,d-d), 7.88 (1H,d-d), 7.68 (1H,tri), 3.92 (3H,s), 3.83 (2H,s) ppm

### <Reference Example 2> Synthesis of 3-hydroxymethyl-2-nitro-phenylacetonitrile

Sodium borohydride (1.51 g, 40.0 mmol) was added at room temperature to a tetrahydrofuran solution (20 ml) of methyl 3-cyanomethyl-2-nitro-phenylbenzoate (4.40 g, 20.0 mmol). To the mixture being refluxed was dropwise added methanol (3.84 g, 0.12 mol) slowly. After the completion of the dropwise addition, refluxing was continued for 1 hour until there was no foaming. After cooling, the reaction mixture was poured into water and extraction with ethyl acetate was made. The organic layer was washed with water, dried over anhydrous magnesium sulfate, and concentrated to obtain a crude product. The crude product was purified using a silica gel column (ethyl acetate:hexane = 1:1) to obtain deep green crystals of 3-hydroxymethyl-2-nitro-phenylacetonitrile (yield: 2.90 g and 76%, melting point: 60 to 62°C).

### <Reference Example 3> Synthesis of (3-bromomethyl-2-nitro-phenyl)acetonitrile

(3-Hyrdoxymethyl-2-nitro-phenyl)acetonitrile (1.7 g, 8.8 mmol) was added to a mixed solvent of toluene (9 ml) and tetrahydrofuran (3 ml) in which phosphorus pentabromide (3.0 g, 9.7 mmol) was dispersed. The mixture was stirred at room temperature for 1 hour. The reaction mixture was poured into ice water and extraction was made with ethyl acetate. The organic layer was washed with water, dried over anhydrous magnesium sulfate, and concentrated to obtain a crude product. The crude product was purified using a silica gel column (ethyl acetate:hexane = 1:5 to 1:2) to obtain light brown crystals of (3-bromomethyl-2-nitro-phenyl)acetonitrile (yield: 1.75 g and 78%) having a melting point of 71 to 73°C.

### Synthesis of (3-methoxymethyl-2-nitro-phenyl)acetonitrile

A methanol solution (20 ml) of (3-bromomethyl-2-nitro-phenyl)acetonitrile (1.3 g, 5.1 mmol) was refluxed for 5 hours. After cooling, the solution was concentrated in vacuo to remove methanol. The residue was poured into water and extraction with ethyl acetate was made. The organic layer was washed with water, dried over anhydrous magnesium sulfate, and concentrated to obtain a crude product. The crude product was purified using a silica gel column (ethyl acetate:hexane = 1:5 to 1:2) to obtain light brown to brown crystals of (3-methoxymethyl-2-nitro-phenyl)acetonitrile (0.2 g, yield: 19%) having a melting point of 34 to 36°C.

### <Reference Example 4> Synthesis of 2-(4,6-dimethoxypyrimidin-2-yl)-2-(3-methoxymethyl-2-nitro-phenyl)acetonitrile

60% sodium hydride (11.2 g, 0.28 mol) was suspended in N,N-dimethylformamide (100 ml). The suspension was cooled to 10°C or less in an ice water bath. Thereto was dropwise added, with stirring, a suspension of (3-methoxymethyl-2-nitro-phenyl)acetonitrile (29 g, 0.14 mol) in N,N-dimethylformamide (100 ml). After the completion of the dropwise addition, stirring was made at room temperature until there was no generation of hydrogen. The reaction mixture was cooled to 10°C or less in an ice water bath. Thereto was added, with stirring, 4,6-dimethoxypyrimidine-2-methylsulfonylpyrimidine (30 g, 0.14 mol), followed by stirring at room temperature for 12 hours. The reaction mixture was poured into ice water. The mixture was made acidic with 10% hydrochloric acid. The crude crystals which appeared, were separated by filtration and washed with water and an ethanol-isopropyl ether mixed solvent to obtain 2-(4,6-dimethoxypyrimidin-2-yl)-2-(3-methoxymethyl-2-nitro-phenyl)acetonitrile (yield: 42 g and 87%) as a reddish brown powder (melting point: 112 to 113°C).

### Confirmed data

- ¹H-NMR 300 MHz CDCl₃ TMS: 7.83 (m,1H), 7.58 (m,2H), 5.91 (s,1H), 5.72 (s,1H), 4.53 (s,2H), 3.90 (s,6H), 3.39 (s,3H)

### <Reference Example 5> Synthesis of (3-methoxymethyl-2-nitro-phenyl)(4,6-dimethoxypyrimidin-2-yl)ketone

In acetic acid (22 ml) was dissolved 1-(4,6-dimethoxypyrimidin-2-yl)-1-(3-methoxymethyl-2-nitro-phenyl) acetonitrile (7.6 g, 22.1 mmol). Thereto were added hydrogen peroxide (4.3 g, 44.2 mmol) and sodium tungstate (0.73 g, 2.2 mmol). The mixture was heated at 70°C for 2 hours to give rise to a reaction. Completion of the reaction was confirmed by TLC (thin layer chromatography), after which acetic acid was distilled off using an evaporator. To remove a trace amount of remaining acetic acid, methanol was added and the mixture was concentrated.

The residue was dissolved in methanol (22 ml) and tetrahydrofuran (22 ml). 25% sodium hydroxide (5.3 g, 33.2 mmol) was added, followed by stirring at room temperature for 1 hour to give rise to a reaction. After the completion of the reaction, ethyl acetate was added. The mixture was washed with water, an aqueous sodium bicarbonate solution and water in this order, dried and concentrated to dryness. The resulting solid was washed with diisopropyl ether to obtain a white powder of (3-methoxymethyl-2-nitro-phenyl)(4,6-dimethoxypyrimidin-2-yl)ketone (yield: 5.8 g and 79%, melting point: 111 to 113°C).

### Confirmed data

- ¹H-NMR (CDCl₃) δ:: 7.90 (1H,d-d), 7.72 (1H,tri), 7.60 (1H,d-d), 6.13 (1H,s), 4.78 (2H,s), 3.90 (6H,s), 3.47 (3H,s) ppm

### <Example 3>

### (D) Synthesis of 3-methoxymethyl-2-nitro-phenylacetaldoxime

There were fed, in a nitrogen atmosphere, 3-methoxymethyl-2-nitro-{2-(pyrrolidin-1-yl)ethenyl}benzene (262.3 g, 1.0 mol) and diisopropyl ether (500 ml). The mixture was cooled in an ice bath. Thereto was added hydroxylamine hydrochloride (90.3 g, 1.3 mol) at 30°C or less. Successively, the mixture was aged at 25 to 30°C for 2 hours. To the reaction mixture was added water (1,300 ml) to conduct extraction. Reextraction was made with diisopropyl ether (300 ml). The diisopropyl ether layers obtained were combined, dried over anhydrous sodium sulfate, and concentrated to obtain intended 3-methoxymethyl-2-nitro-phenylacetaldoxime (213.0 g, 0.95 mol, yield: 95.0%).

### <Example 4>

### (D) Synthesis of 3-methoxymethyl-2-nitro-phenylacetoaldoxime

In the same operation as in Example 3 except that diisopropyl ether (500 ml) was changed to toluene (500 ml), there was obtained intended 3-methoxymethyl-2-nitro-phenylacetaldoxime (215.3 g, 0.96 mol, yield: 96.0%).

### Industrial Applicability

According to the present process, there can be produced industrially easily a (2-nitro-phenyl)acetonitrile derivative represented by the general formula [IV], which becomes a useful intermediate for agricultural chemicals described in, for example, WO 00/06553. Also, according to the present invention, there is provided a useful intermediate for agricultural chemicals described in WO 00/06553.

## Claims

1. A process for producing a (2-nitropneyl)acetonitrile derivative represented by the following general formula [IV] 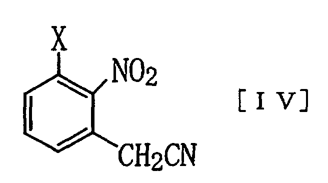 (wherein X is an alkoxymethyl group or an alkoxycarbonyl group), which comprises the following steps (A) to (C).
(1) A step (A) which comprises reacting a 2-methylnitrobenzene derivative represented by the following general formula [I] 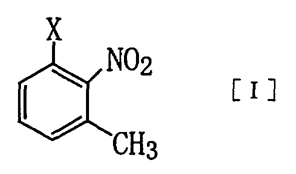 (wherein X is an alkoxymethyl group or an alkoxycarbonyl group) with an N,N-dialkylformamide dialkylacetal represented by the following general formula [VI]
(R₁) (R₂)N-CH(OR₃) (OR₄) [VI]
(wherein R₁ and R₂ may be the same or different, are each an alkyl group, and may form, by bonding to each other, a 5- to 6-membered ring together with the nitrogen atom; and R₃ and R₄ may be the same or different and are each an alkyl group), or with a mixture of N,N-dimethylformamide dimethylacetal represented by the following formula [VII]
(CH₃)₂N-CH(OCH₃)₂ [VII]
and a dialkylamine represented by the following general formula [VIII]
(R₁) (R₂)NH [VIII]
(wherein R₁ and R₂ have the same meanings as given above) to obtain a 2-(2-dialkylaminoethenyl)nitrobenzene derivative represented by the following general formula [IX] 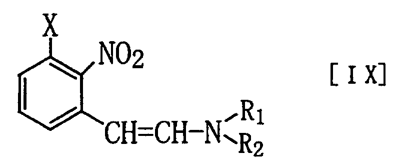 (wherein X, R₁ and R₂ have the same meanings as given above), and hydrolyzing the derivative [IX] under acidic conditions to obtain a phenylacetoaldehyde derivative represented by the following general formula [II] 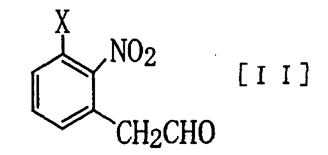 (wherein X has the same meaning as given above).
(2) A step (B) which comprises reacting the phenylacetoaldehyde derivative represented by the general formula [II], obtained in the step (A) with hydroxylamine to obtain a phenylacetoaldoxime derivative represented by the following general formula [III] 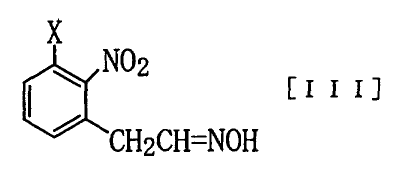 (wherein X has the same meaning as given above).
(3) A step (C) which comprises dehydrating the phenylacetoaldoxime derivative represented by the general formula [III], obtained in the step (B) to obtain a (2-nitro-phenyl)acetonitrile derivative represented by the following general formula [IV] 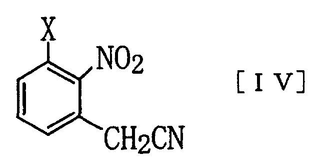 (wherein X has the same meaning as given above).

2. A process for producing a (2-nitro-phenyl)acetonitrile derivative represented by the following general formula [IV] 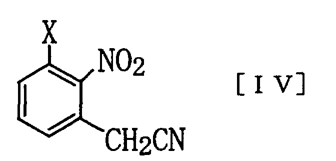 (wherein X is an alkoxymethyl group or an alkoxycarbonyl group), which comprises the following steps (D) and (E).
(1) A step (D) which comprises reacting a 2-methylnitrobenzene derivative represented by the following general formula [I] 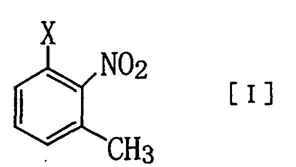 (wherein X is an alkoxymethyl group or an alkoxycarbonyl group) with an N,N-dialkylformamide dialkylacetal represented by the following general formula [VI]
(R₁) (R₂)N-CH(OR₃) (OR₄) [VI]
(wherein R₁ and R₂ may be the same or different, are each an alkyl group, and may form, by bonding to each other, a 5- to 6-membered ring together with the nitrogen atom; and R₃ and R₄ may be the same or different and are each an alkyl group), or with a mixture of N,N-dimethylformamide dimethylacetal represented by the following formula [VII]
(CH₃)₂N-CH(OCH₃)₂ [VII]
and a dialkylamine represented by the following general formula [VIII]
(R₁) (R₂)NH [VIII]
(wherein R₁ and R₂ have the same meanings as given above) to obtain a 2-(2-dialkylaminoethenyl)nitrobenzene derivative represented by the following general formula [IX] 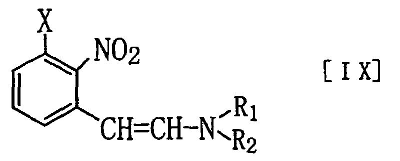 (wherein X, R₁ and R₂ have the same meanings as given above), and reacting the derivative [IX] with hydroxylamine to obtain a phenylacetoaldoxime derivative represented by the following general formula [III] 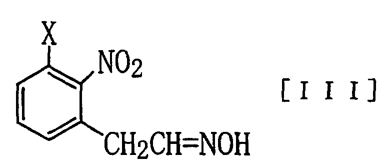 (wherein X has the same meaning as given above).
(2) A step (E) which comprises dehydrating the phenylacetoaldoxime represented by the general formula [III], obtained in the step (D) to obtain a (2-nitro-phenyl)acetonitrile derivative represented by the following general formula [IV] 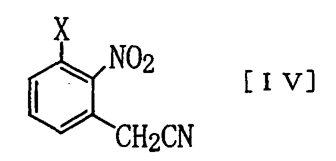 (wherein X has the same meaning as given above).

3. A process for producing a phenylacetoaldoxime derivative represented by the following general formula [III] 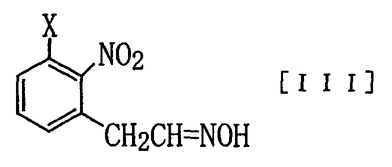 (wherein X is an alkoxymethyl group or an alkoxycarbonyl group), which comprises reacting a 2-(2-dialkylaminoethenyl)nitrobenzene derivative represented by the following general formula [IX] 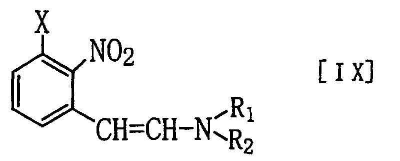 (wherein X has the same meaning as given above; and R₁ and R₂ may be the same or different, are each an alkyl group, and may form, by bonding to each other, a 5- to 6-membered ring together with the nitrogen atom) with hydroxylamine.

4. A nitrobenzene derivative represented by the following general formula [V] 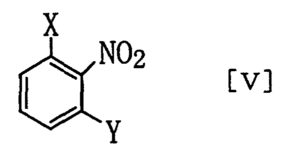 [wherein X is an alkoxymethyl group or an alkoxycarbonyl group; Y is a methyl group, a 2-dialkylaminoethenyl group represented by the following general formula [XI]
-CH=N(R₁)(R₂) [XI]
(wherein R₁ and R₂ may be the same or different, are each an alkyl group, and may form, by bonding to each other, a 5- to 6-membered ring together with the nitrogen atom), a formylmethyl group or a 2-hydroxyiminoethyl group; when Y is a methyl group or a 2-dialkylaminoethenyl group represented by the general formula [XI], X is a methoxymethyl group].
